# EUROPÄISCHE PATENTSCHRIFT

(11) **EP 2 417 201 B1**
(45) Veröffentlichungstag und Bekanntmachung des Hinweises auf die Patenterteilung: **12.06.2013**
(21) Anmeldenummer: 10718506.8
(22) Anmeldetag: 01.04.2010
(51) Int. Cl.: C09B 57/10, H01L 51/50, C09K 11/06

(54) **FARBSTOFFE AUF BASIS VON BISTERPYRIDINE-METALLKOMPLEXVERBINDUNGEN**
DYES BASED ON BIS-TERPYRIDINE METAL COMPLEX COMPOUNDS
COLORANTS À BASE DE COMPOSÉS COMPLEXES MÉTAL- BISTERPYRIDINE

(30) Priorität: 08.04.2009 DE 102009003767
(43) Veröffentlichungstag der Anmeldung: 15.02.2012
(73) Patentinhaber: Karlsruher Institut für Technologie, 73131 Karlsruhe (DE)
(72) Erfinder: SCHRAMM, Frank, 76139 Karlsruhe (DE); RUBEN, Mario, 67000 Strasbourg (FR); MEDED, Velimir, 68167 Mannheim (DE); EVERS, Ferdinand, 76297 Stutensee (DE)
(74) Vertreter: Fitzner, Uwe
(86) Internationale Anmeldenummer: PCT/EP2010/054438
(87) Internationale Veröffentlichungsnummer: WO 2010/115848

(56) Entgegenhaltungen:
- EP-A1- 2 036 955
- M.MAESTRI ET AL.: "Complex of the Ruthenium(II)-2,2':6',2''-Terpyridine Family" INORG.CHEM., Bd. 34, 1995, Seiten 2759-2767, XP002597635 in der Anmeldung erwähnt
- M.ABRAHAMSSON ET AL.: "A 3.0 microsec. Room Temperature Excited State Lifetime of a Bistridentate RuII-Polypyridine Complex for Rod-like Molecular Arrays" JACS, Bd. 128, 2006, Seiten 12616-12617, XP002597636
- H.WOLPHER ET AL.: "A tridentate Ligand for Preparation of Bisterpyridine-like Ruthenium(II) complexes with increased exited State Lifetime" INOR.CHEM.COMM., Bd. 7, 2004, Seiten 337-340, XP002597637
- M.ABRAHAMSSON ET AL.: "Bistridentate Ruthenium(II)polypyridyl-Type Complexes with Microsecond 3MLCT State Litetimes" JACS, Bd. 130, 2008, Seiten 15533-15542, XP002597638
- X.-D. CHEN ET AL.: "Solvent-controlled generation of two Pairs of mononuclear single-stranded helical Copper(II) Complexes of 2,6-Pyridinediylbis(2-pyridinyl)methanone" INORG.CHIM.ACTA, Bd. 358, 2005, Seiten 1107-1112, XP002597639 in der Anmeldung erwähnt
- B.ABARCA ET AL.: "A facile Route to new potential helicating Ligands" TETRAHEDRON, Bd. 54, 1998, Seiten 15287-15292, XP002597640

## Beschreibung

Die vorliegende Erfindung betrifft einen Farbstoff, ein Verfahren zu dessen Herstellung, sowie dessen Verwendung.

### Stand der Technik:

Bis-Terdentat-Metallkomplexe auf Basis der (substituierten) 2,2':6',2"-Terpyridine sind bekannt, jedoch sind deren Photolumineszenzeigenschaften nicht ausreichend für potentielle photokatalytische Umsetzungsprozesse in Gegenwart von Sauerstoff und bei Raumtemperatur. Das Funktionieren unter natürlichen, bzw. naturnahen, Umgebungsumständen ist für ein effektiv arbeitendes Photokatalysatorsystem essenziell. Ein Weg zur Verbesserung der Lichtaktivitätseigenschaften ist die Erweiterung des Bisswinkels des Liganden, das zum Beispiel durch die formale Einführung eines Kohlenstoffatoms zwischen zwei Pyridinuntereinheiten realisiert werden kann. Terpyridinsysteme, wie Bis-2,2':6',2"'-terpyridin-Ruthenium(II)-komplexe {Ru(tpy)₂} oder Ru(tpy)₂(PF₆)₂ sind in M. Maestri, N. Armaroli, V. Balzani, E. C. Constable, A. M. W. C. Thompson, Inorg. Chem. 1995, 34, 2759 beschrieben. Einzel-Ligand Metallkomplexverbindungen wie Mono-Di-2',6'-(2,2"-Nicotinyl)pyridine)-Kupfer(II)-Dichlorid (pyCOpyCOpy)CuCl₂ sind in X. D. Chen, T. C.W. Mak Inorg. Chim. Acta 2005, 358, 1107 beschrieben. Diese haben strukturell nur einen dreizähnigen Liganden, und sind demnach von trigonalbipyramidaler Koordinationsgeometrie. Außerdem ist {(pyCOpyCOpy)CuCl₂ nicht photolumineszent und kommt demnach nicht als Photokatalysator in Betracht.

Einseitig Carbonyl-insertierte Terpyridin-Liganden wie Bis-(2,2'-bipyridyl-6-carbonyl-2"-pyridine)-Ruthenium(II)-komplexe {Ru(bpyCOpy)₂} sind aus T. Bark, R. P. Thummel, Inorg. Chem. 2005, 44, 8733 bekannt.

Die (spontane) Oxidation des Liganden in Gegenwart von Sauerstoff wurde bereits S. Rau, M. Schwalbe, S. Losse, H. Görls, C. McAlister, F. M. MacDonnell, J. G. Vos Eur. J. Inorg. Chem. 2008, 1031 beschrieben.

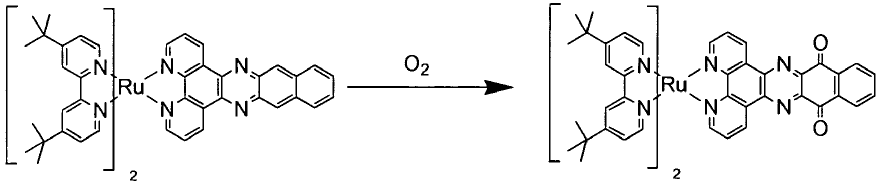

Allerdings wurde in der genannten Arbeit explizit Licht als Aktivator eingesetzt.

Aus M. Abrahamsson et al. JACS, Bd. 128, 2006, Seiten 12616-12617 sind Ruthenium-Komplexe (Ru(bqp)₂) bekannt, wobei Quinolinyl-Teilstrukturen durch Methylengruppen verbrückt sind. Ähnliche Offenbarungen finden sich in Abrahamsson et al. JACS, Bd. 130, 2008, Seiten 15533-15542 und Wolpher et al. Inor. Chem. Comm., Bd. 7, 2004, Seiten 337-240 bekannt.

Die Verbindungen des Standes der Technik weisen alle eine zu geringe Quantenausbeute der Photolumineszenz auf und/oder sind gegenüber der Anwesenheit von Sauerstoff zu empfindlich.

Aus Abarca et al. Tetrahedron, Bd. 54, 1998, Seiten 15287-15292 sind Terpyridin-Strukturen und Kupfer-Komplexe davon bekannt.

### Aufgabe:

Aufgabe der vorliegenden Erfindung ist es Verbindungen, insbesondere Metallkomplexe, zur Verfügung zu stellen, die verbesserte Photolumineszenzeigenschaften, insbesondere erhöhte Quantenausbeuten und erhöhte Stabilität gegenüber Sauerstoff, insbesondere unter gleichzeitiger Lichteinwirkung aufweisen. Die neuen Verbindungen sollen leicht und mit guten Ausbeuten herstellbar sein und sich für vielfältige Anwendungszwecke eignen.

Ferner sollte ein geeignetes Verfahren zu deren Herstellung gefunden werden, sowie adäquate Verwendungszwecke für die neuen Verbindungen.

### Lösung:

Diese Aufgabe wird durch die Verbindungen der allgemeinen Formel 1 gelöst, sowie durch das erfindungsgemäße Verfahren zu deren Herstellung und deren Verwendung.

### Begriffsdefinitionen:

Im Rahmen der vorliegenden Erfindung sind alle Mengenangaben, sofern nicht anders angegeben, als Gewichtsangaben zu verstehen.

Im Rahmen der vorliegenden Erfindung bedeutet der Begriff "Zimmertemperatur" eine Temperatur von ca. 20°C; der Begriff "Raumtemperatur" eine Temperatur von ca. 23°C. Temperaturangaben sind, soweit nicht anders angegeben, in Grad Celsius (°C).

Sofern nichts anderes angegeben wird, werden die angeführten Reaktionen bzw. Verfahrensschritte bei Normaldruck (Atmosphärendruck) durchgeführt.

### Detaillierte Beschreibung:

Ein Gegenstand der vorliegenden Erfindung sind Verbindungen der allgemeinen Formel 1 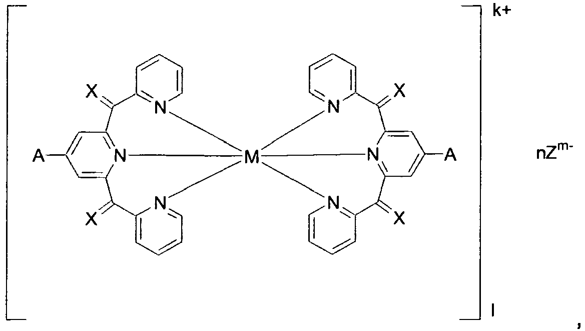 worin
- A =: jegliche Art der Substitution, bevorzugt ausgewählt aus der Gruppe bestehend aus organischen Substituenten, -CF₃, -F, -Cl, -Br, -I, -OH, -SH, -NH₂, -NO₂ oder -H,
wobei die organischen Substituenten bevorzugt ausgewählt werden aus der Gruppe bestehend aus
- Arylresten, insbesondere Phenyl-, Anthracenyl-, Pyrenyl-,
- heteroaromatischen Resten, insbesondere Pyridyl-, Nicotinyl-, Picolyl-,
- ungesättigten Kohlenwasserstoffresten, wie C₂-C₆-Alkinyl-, C₂-C₁₀-Alkenyl-, Allyl-, Benzyl-, und
- gesättigten Kohlenwasserstoffresten wie C₁-C₂₀-Alkyl-,
- -OR, -SR, -B(OR)₂, -COOR, -NHR, -NR₂, -CHR
wobei R für Aryl- und C₁-C₂₀-Alkyl- steht, insbesondere für Phenyl-, Pyridyl oder C₁-C₁₀-Alkyl
- X =: O, S, NR, NH, PR, PH, wobei R für Aryl- und C₁-C₂₀-Alkyl- steht, insbesondere für Phenyl- oder C₁-C₁₀-Alkyl
- M =: Übergangsmetall in einer Oxidationsstufe ausgewählt aus der Gruppe bestehend aus 0, +1, +2, +3, +4, +5, +6, +7 und +8, bevorzugt aus der Gruppe bestehend aus +1, +2, +3 und +4. Bevorzugt steht M für Übergangsmetallionen ausgewählt aus der Gruppe bestehend aus Cr³⁺, Mn²⁺, Re⁺, Re³⁺, Fe²⁺, Fe³⁺, Ru²⁺, Ru³⁺, Os²⁺, Co²⁺, Co³⁺, Rh⁺, Rh³⁺, Ir³⁺, Ni²⁺, Ni³⁺, Pd²⁺, Pt²⁺ und Pt⁴⁺
- Z =: Gegenion mit m-facher negativer Ladung, bevorzugt wird Z ausgewählt aus der Gruppe bestehend aus BF₄⁻, ClO₄⁻, SO₄²-, PO₄³⁻, F⁻, Cl⁻, Br, I⁻, NO³⁻, PF₆⁻, O²⁻, OH⁻, CO₃²⁻, CN⁻, CF₃SO₃⁻, Cyanat, Isocyanat, Thiocyanat, Isothiocyanat, Tetraphenylborat und Acetat.
- k: k-fache Ladung; nichtnegative ganze Zahl, bevorzugt k = 0, 1, 2, 3, 4, 5, 6, 7, 8, besonders bevorzugt k = 1, 2, 3, 4, insbesondere bevorzugt k = 1, 2, 3,
- l: molekulares Äquivalent; positive ganzen Zahlen (1, 2, 3...),
- m: m-fache negative Ladung; positive ganze Zahl, bevorzugt m = 1, 2, 3 insbesondere bevorzugt m = 1, 2,
- n: molekulares Äquivalent; nichtnegative ganzen Zahlen (0, 1, 2, 3...) und
mit der Maßgabe, dass die Bedingung k*l=n*m erfüllt ist.

Dabei sind die Werte für I und n jeweils so anzupassen, dass das Gesetz der Ladungsneutralität (k*l=n*m) erfüllt ist, wobei sich deren jeweiliger Zahlenwert für den Fachmann ohne weiteres ergibt.

Im Rahmen der vorliegenden Erfindung ist es möglich, dass an den beiden Terpyridin-Liganden die Substituenten "A" jeweils gleich oder unterschiedlich sind.

Im einer Alternative der vorliegenden Erfindung ist es auch möglich, das verschiedene Gegenionen Z verwendet werden.

Höchst bevorzugt haben im Rahmen der vorliegenden Erfindung die Variablen die folgenden Bedeutungen:
- A =: Benzyl-, Phenyl-, Ethinyl-, -F, -OH, -NH₂, -H, insbesondere Wasserstoff,
- X =: O, S, NH, insbesondere O,
- M =: ausgewählt aus der Gruppe bestehend aus Cr³⁺, Mn²⁺, Re⁺, Re³⁺, Fe²⁺, Fe³⁺, Ru²⁺, Ru³⁺, Os²⁺, Co²⁺, Co³⁺, Rh⁺, Rh³⁺, Ir³⁺, Ni²⁺, Ni³⁺, Pd²⁺, Pt²⁺ und Pt⁴⁺, insbesondere Ru²⁺ und Ir³⁺,
- Z =: ausgewählt aus der Gruppe bestehend aus BF₄⁻, ClO₄⁻, SO₄²⁻, PO₄³⁻, F⁻, Cl⁻, Br, I⁻, NO³⁻, PF₆⁻, O²⁻, OH-, CO₃²⁻, CN⁻, CF₃SO₃⁻, Cyanat, Isocyanat, Thiocyanat, Isothiocyanat, Tetraphenylborat und Acetat, insbesondere PF₆⁻.

In der am allermeisten bevorzugten Variante der vorliegenden Erfindung ist A = Wasserstoff und X = Sauerstoff, der Ligand also 2',6'-(Di(2:2"-pyridylcarbonyl)pyridin.

In der am allermeisten bevorzugten Variante der vorliegende Erfindung sind die Übergangsmetalle Ruthenium in der Oxidationsstufe +2 oder Iridium in der Oxidationsstufe +3.

Die am meisten bevorzugte Verbindung im Rahmen der vorliegenden Erfindung ist Bis[2',6'-di-(2:2"-pyridylcarbonyl)pyridin]ruthenium(II)-Hexafluorophosphat (Verbindung 1).

Eine weitere sehr bevorzugte Verbindung im Rahmen der vorliegenden Erfindung ist die analoge Iridium(III)-Verbindung Bis[2',6'-di-(2:2"-pyridylcarbonyl)pyridin]iridium(III)-Hexafluorophosphat (Verbindung 2).

Weiterhin ist Gegenstand der vorliegenden Erfindung ein Verfahren zur Herstellung von Bis[2',6'-di-(2:2"-pyridylcarbonyl)pyridin]ruthenium(II)-Hexafluorophosphat oder Bis[2',6'-di-(2:2"-pyridylcarbonyl)pyridin]iridium(III)-Hexafluorophosphat umfassend die Schritte:
a) Auflösen von Ru(DMSO)₄Cl₂, oder Ir(DMSO)₄Cl₄ in Dimethylformamid,
b) Zugabe von 2,6-Bis(2-picolinyl)pyridin,
c) Erhitzen der Mischung für 240 bis 480 Minuten auf 90 bis 110°C,
d1) Abkühlen oder Abkühlen lassen, und
d2) Entfernung des Lösungsmittels, Aufreinigung des Reaktionsprodukts, durch Chromatographie, mit Acetonitril/Wasser/gesättigter KNO₃-Lösung im Volumenverhältnis 80:20:2 als Eluationsmittel, neuerliches Lösen des Feststoffs mit Methanol,
e) Versetzen der Lösung aus Schritt d2) mit wässriger Ammonium-Hexafluorophosphat-Lösung,
f) Abfiltrieren und Aufarbeiten des ausfallenden Niederschlags.

Ein bevorzugtes Verfahren der vorliegenden Erfindung ist ein Verfahren zur Herstellung von Bis[2',6'-di-(2:2"-pyridylcarbonyl)pyridin]ruthenium(II)-Hexafluorophosphat
umfassend die Schritte:
a) Auflösen von Ru(DMSO)₄Cl₂ in Dimethylformamid,
b) Zugabe von 2,6-Bis(2-picolinyl)pyridin,
c) Erhitzen der Mischung für 10 Stunden auf 100°C,
d1) Abkühlen oder Abkühlen lassen, und
d2) Entfernung des Lösungsmittels, Aufreinigung des Reaktionsprodukts, durch Chromatographie, mit Acetonitril/Wasser/gesättigter KNO₃-Lösung im Volumenverhältnis 80:20:2 als Eluationsmittel, neuerliches Lösen des Feststoffs mit Methanol,
e) Versetzen der Lösung aus Schritt d2) mit wässriger Ammonium-Hexafluorophosphat-Lösung,
f) Abfiltrieren und Aufarbeiten des ausfallenden Niederschlags.

Nicht zuletzt ist die Verwendung der Verbindungen der allgemeinen Formel 1 und der nach dem erfindungsgemäßen Verfahren hergestellten Verbindungen in photovoltaischen Zellen bzw. Solarzellen, als Photokatalysator, als Sensibilisator für chemische Reaktionen, insbesondere in der Synthesechemie oder als Farbstoff Gegenstand der vorliegenden Erfindung.

Es ist im Rahmen der vorliegenden Erfindung möglich, anstelle einzelner erfindungsgemäßer Verbindungen bei den erfindungsgemäßen Verwendungen auch verschiedene erfindungsgemäße Verbindungen in Kombination einzusetzen, insbesondere bevorzugt ist dabei die Kombination der Verbindungen 1 und 2.

Die Verbindungen der allgemeinen Formel 1 haben stärker oktaedrisch aufgebaute Koordinationsumgebung als beispielsweise Ru(tpy)₂-Verbindungen oder Ru(bpyCOpy)₂-Verbindungen. Die Verbindungen der allgemeinen Formel 1 weisen wesentlich längere Lebenszeiten des angeregten Zustandes und bedeutend höhere Quanteneffizienz der Photolumineszenz, insbesondere bei Raumtemperatur, als Bis-Terpyridin-Ruthenium-Systeme oder Bis-(2,2'-bipyridyl-6-carbonyl-2'-pyridin)-Ruthenium(II) auf. Der angeregte Zustand der Verbindungen der allgemeinen Formel 1 zeigt eine wesentlich höhere Resistenz gegenüber dem Quenching mit Sauerstoff, als dies bei Verbindungen gemäß dem Stand der Technik der Fall ist.
Die Verbindungen der allgemeinen Formel 1 besitzen höhere Symmetrie und weisen keine Stereoisomeren auf, im Gegensatz zu Ru(bpyCOpy)₂.

Bei der Synthese der Verbindungen der allgemeinen Formel 1 wurde kein Unterschied in der Reaktivität in der Gegenwart von Tageslicht festgestellt. Die Verbindungen der allgemeinen Formel 1 unterliegen also keiner lichtabhängigen Ligandenoxidation in Gegenwart von Sauerstoff und Tageslicht.

Die Verbindungen der allgemeinen Formel 1 weisen zwei pyCOpyCOpy Liganden auf und sind von nahezu ideal oktaedrischer Koordinationsgeometrie. Bei den Verbindungen der allgemeinen Formel 1 agieren im Ligandengerüst zwei Carbonylgruppen zwischen den Pyridinringen als Platzhalter, die die pi-Konjugation des gesamten Ligandsystems erhalten.

Die erfindungsgemäßen Verbindungen sind Farbstoffe, die insbesondere auch fluoreszente und/oder phosphoreszente Eigenschaften aufweisen.

Die Verbindungen gemäß vorliegender Erfindung weisen außerordentlich lange Lebenszeiten des angeregten Zustandes und hohe Quantenausbeuten der Photolumineszenz auf. Das ist wichtig für die Herstellung von hocheffizienten Photovaltaikzellen.

Vorteile der Verbindungen gemäß vorliegender Erfindung sind weiter, dass längere Lebenszeiten des angeregten Zustandes erreichbar sind, die eine größere Variation potentiell anregbarer Moleküle erlauben. Die hohe Resistenz gegenüber Sauerstoff als Quencher erlaubt gezielte Bevorzugung geeigneter Reaktionspartner in Energieübertragungsprozessen.

Nicht zuletzt Gegenstand der vorliegenden Erfindung sind photovoltaische Zellen oder Solarzellen enthaltend eine oder mehrere, bevorzugt eine, Verbindung(en) der allgemeinen Formel 1, insbesondere die Verbindungen 1 und/oder 2.

Die verschiedenen Ausgestaltungen der vorliegenden Erfindung, z.B. diejenigen der verschiedenen abhängigen Ansprüche, können dabei in beliebiger Art und Weise miteinander kombiniert werden.

Die Erfindung wird nun unter Bezugnahme auf die folgenden nichtlimitierenden Beispiele erläutert.

### Beispiele:

### Beispiel 1:

### Herstellung von Bis[2',6'-di(2:2"-pyridylcarbonyl)pyridin]ruthenium(II)-di(hexafluorophosphat) 1:

100 mg (206 µmol) Ru(DMSO)₄Cl₂ wurde in einem Volumen von 30 ml Dimethylformamid vollständig aufgelöst. Danach wurden 135 mg (515 µmol) 2,6-Bis(2-picolinyl)pyridin zugegeben und die Mischung für 10 Stunden auf 100°C erhitzt. Danach wurde auf Raumtemperatur abgekühlt und das Lösungsmittel entfernt.

Das feste Reaktionsprodukt wurde über Säulenchromatographie auf SiO₂ gereinigt, das erste rotgefärbte Band eluiert, und das Elutionsmittel (Acetonitril/Wasser/KNO₃-Lösung mit Volumenanteilen 80:20:2) entfernt.

Der rote Feststoff wurde dann mit Methanol vom Hilfssalz Kaliumnitrat gelöst und die Lösung anschließend mit wässriger Ammonium-Hexafluorophosphat-Lösung versetzt. Der dann ausgefallene Niederschlag wurde abfiltriert und der tiefrote Feststoff in Aceton gelöst und mit wenig Wasser und Methanol versetzt. Die organischen Lösungsmittel wurden langsam durch Verdunsten bei Raumtemperatur und Normaldruck in einem offenen Glasgefäß verdampft und die anfallenden tiefdunkelroten Kristalle abfiltriert.

Es wurden 16 mg (20 µmol) der Verbindung 1 erhalten, was einer Ausbeute von 10% entsprach.

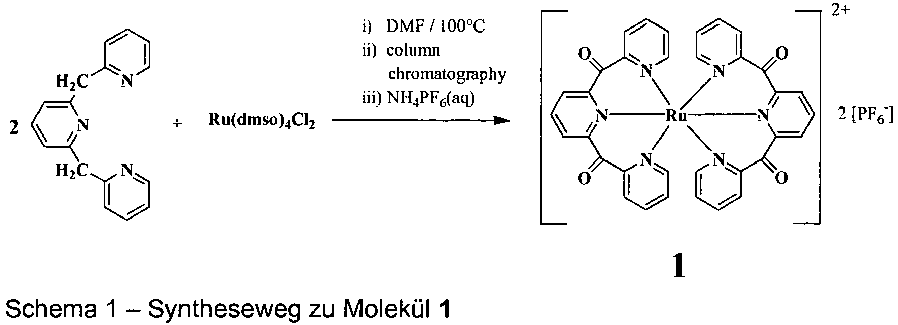

Die Synthese von **1** gelang durch Verwendung des pyCH₂pyCH₂py Liganden nach oben aufgeführtem Weg.

### Beispiel 2 (Vergleichsbeispiel):

Alternativer Versuch zur Herstellung von Molekül 1. Dazu wurde versucht, das Molekül 1 auf direkten Weg aus dem pyCOpyCOpy-Liganden mit Ru(dmso)₄Cl₂ zu synthetisieren.

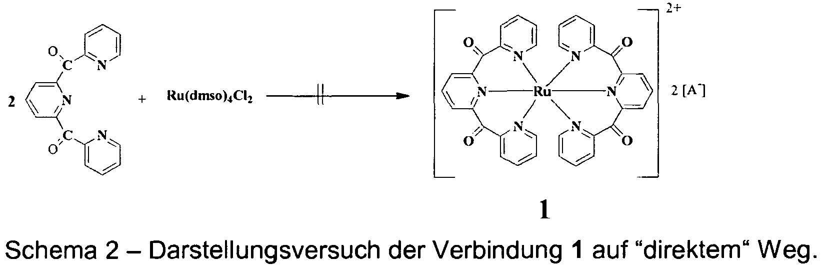

Die Synthese des Moleküls auf direkten Weg aus dem pyCOpyCOpy-Liganden mit Ru(dmso)₄Cl₂ gelang nicht.

### Vergleichsdaten:

In der folgenden Tabelle sind ausgewählte photophysikalische Eigenschaften einer Verbindung des Standes der Technik (Ru(tpy)₂) denen der erfindungsgemäßen Verbindung 1 gegenübergestellt.

**Tabelle 1**

| | | Ru(tpy)₂ | | | Molekül 1 | | |
|---|---|---|---|---|---|---|---|
| | | tₑₘ | Fₑₘ | Iₑₘ | tₑₘ | Fₑₘ | Iₑₘ |
| RT | A | - | - | - | 1.36 | 0.13 | 608 |
| | D | 2.5*10⁻⁴ (a) | <5*10⁻⁶ (c) | 629 (a) | 3.30 | 0.3 | 608 |
| 77 K | A | - | - | - | 6.17 | 0.41 | 613 |
| | D | 10.6 (a) | 0.48 (d) | 598 (a) | 6.43 | 0.43 | 613 |

In der Tabelle 1 bedeuten:
RT = Raumtemperatur
A in Gegenwart von Luft(sauerstoff) gemessen
D unter Schutzgas Argon / unter Ausschluss von Luftsauerstoff gemessen
tₑₘ = Lebenszeit des angeregten Tripletzustandes in Mikrosekunden [µs]
Fₑₘ = Quanteneffizienz der Lumineszenz [dimensionslos]
Iₑₘ = Wellenlänge in Nanometern [nm]
("t", "F" und "I" ersetzen dabei aus drucktechnischen Gründen die eigentlich korrekten griechischen Buchstaben "kleines Tau", Großes Phi" und "kleines Lambda")

Die Werte für Ru(tpy)₂ stammen aus folgenden Literaturstellen:
(a) = M. Maestri, N. Armaroli, V. Balzani, E. C. Constable, A. M. W. C. Thompson, Inorg. Chem. 1995, 34, 2759
(b) = Collin, J.P.; Beley, M.; Sauvage, J.P.; Barigelleti, F. Inorg. Chim. Acta, 1991, 186, 91
(c) = Winkler, J.R.; Netzel, T.L.; Creutz, C.; Sutin, N. J. Am. Chem. Soc. 1987, 109, 2381

### Sauerstoffabhängigkeit der Emissionseigenschaften

Die Emission der erfindungsgemäßen Verbindungen weist eine schwache Abhängigkeit der Intensität, der Lebenszeit und auch der Quantenausbeute von der Gegenwart von (Luft)Sauerstoff auf. Jedoch ist die Abhängigkeit wesentlich geringer als bei anderen Molekülen (siehe Tabelle 1). Die Geschwindigkeitskonstante der strahlungslosen Deaktivierung gibt eine direkte Abhängigkeit vom Sauerstoffpartialdruck wieder. Für Molekül 1 ist die Abhängigkeit der Emission von Sauerstoff geringer als von anderen Systemen.

**Tabelle 2 - Abhängigkeit der Emission e) von der Gegenwart von Luftsauerstoff**

| | | | |
|---|---|---|---|
| kᵣ = Geschwindigkeitskonstante der Licht emittierenden (radiativen) Deaktivierung des angeregten Zustandes; | | | |
| kₙᵣ = Geschwindigkeitskonstante der strahlungslosen (nicht-radiativen) Deaktivierung des emittierenden Zustandes. | | | |

| | e) | kᵣ (s⁻¹) | kₙᵣ(s⁻¹) |
|---|---|---|---|
| Raumtemperatur | Luft | 9.6x10⁴ | 6.4x10⁵ |
| | Argon | 9.1 x10⁴ | 2.1x10⁵ |
| 77 K | Luft | 6.65 x10⁴ | 9.6x10⁴ |
| | Argon | 6.65 x10⁴ | 8.9x10⁴ |

Die Quantenausbeuten der Lumineszenz bei Raumtemperatur, sowohl unter Argon als auch in Gegenwart von Luft sind höher als bei den Vergleichssystemen des Standes der Technik (Siehe Tabelle 1). Die Lebenszeit des angeregten Zustandes in Gegenwart von Luftsauerstoff ist höher als bei den Vergleichssystemen (Tabelle 1). Das Oxidationspotential der Verbindung **1** liegt bei +1.525 V gegenüber dem Silber/Silberchlorid-Elektrodenpaar.

## Patentansprüche

1. Verbindung der allgemeinen Formel 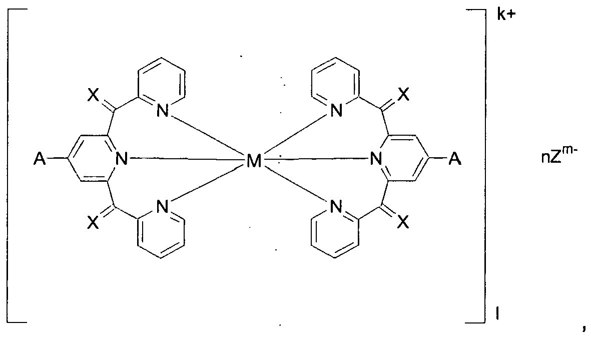 worin
A jegliche Art der Substitution,
X O, S, NR, NH, PR; PH,
M Übergangsmetall mit k-facher Ladung,
Z Gegenion mit m-facher negativer Ladung,
k k-fache Ladung; nichtnegative ganze Zahl,
l molekulares Äquivalent; positive ganze Zahl,
m m-fache negative Ladung; positive ganze Zahl,
n molekulares Äquivalent; nichtnegative ganze Zahl und
mit der Maßgabe, dass die Bedingung k*l=n*m erfüllt ist.

2. Verbindung nach Anspruch 1, **dadurch gekennzeichnet, dass** die Variablen die folgenden Bedeutungen haben
A = ausgewählt aus der Gruppe bestehend aus organischen Substituenten, -CF₃, -F, -Cl, -Br, -I, -OH, -SH, -NH₂, -NO₂ oder -H, wobei die organischen Substituenten bevorzugt ausgewählt werden der Gruppe bestehend aus
- Arylresten, insbesondere Phenyl-, Anthracenyl-, Pyrenyl-,
- heteroaromatischen Resten, insbesondere Pyridyl-, Nicotinyl-, Picolyl-,
- ungesättigten Kohlenwasserstoffresten, wie C₂-C₆-Alkinyl-, C₂-C₁₀-Alkenyl-, Allyl-, Benzyl-, und
- gesättigten Kohlenwasserstoffresten wie C₁-C₂₀-Alkyl-,
- -OR, -SR, -B(OR)₂, -COOR, -NHR, -NR₂, -CHR wobei R für Aryl- und C₁-C₂₀-Alkyl- steht, insbesondere für Phenyl-, Pyridyl oder C₁-C₁₀-Alkyl
X = O, S, NR, NH, PR, PH, wobei R für Aryl- und C₁-C₂₀-Alkyl- steht, insbesondere für Phenyl- oder C₁-C₁₀-Alkyl,
M = Übergangsmetall, in einer Oxidationsstufe ausgewählt aus der Gruppe bestehend aus 0, +1, +2, +3, +4, +5, +6, +7 und +8, bevorzugt aus der Gruppe bestehend aus +1, +2, +3 und +4, insbesondere bevorzugt ausgewählt aus der Gruppe bestehend aus Cr³⁺, Mn²⁺, Re⁺, Re³⁺, Fe²⁺, Fe³⁺, Ru²⁺, Ru³⁺, Os²⁺, Co²⁺, Co³⁺, Rh⁺, Rh³⁺, Ir³⁺, Ni²⁺, Ni³⁺, Pd²⁺, Pt²⁺ und Pt⁴⁺,
Z = Gegenion mit m-facher negativer Ladung, bevorzugt ausgewählt aus der Gruppe bestehend aus BF₄⁻, ClO₄⁻, SO₄²⁻, PO₄³⁻, F⁻, Cl⁻, Br, I⁻, NO³⁻, PF₆⁻, O²⁻, OH⁻, CO₃²⁻, CN-, CF₃SO₃⁻, Cyanat, Isocyanat, Thiocyanat, Isothiocyanat, Tetraphenylborat und Acetat,
k k-fache positive Ladung; nichtnegative ganze Zahl k = 0, 1, 2, 3, 4, 5, 6, 7, 8, bevorzugt 1, 2, 3, 4, insbesondere bevorzugt 1, 2, 3.
l molekulares Äquivalent; positive ganze Zahl,
m m-fache negative Ladung; positive ganze Zahl m = 1, 2, 3, bevorzugt 1, 2, insbesondere bevorzugt 1,
n molekulares Äquivalent; nichtnegative ganze Zahl und
mit der Maßgabe, dass die Bedingung k*l=n*m erfüllt ist.

3. Verbindung nach Anspruch 1 oder 2, **dadurch gekennzeichnet, daß** die Variablen die folgenden Bedeutungen haben
A = Benzyl-, Phenyl-,Ethinyl-, -F, -OH, -NH₂, -H, insbesondere Wasserstoff,
X = O, S, NH, insbesondere O,
M = ausgewählt aus der Gruppe bestehend aus Cr³⁺, Mn²⁺, Re⁺, Re³⁺, Fe²⁺, Fe³⁺, Ru²⁺, Ru³⁺, Os²⁺, Co²⁺, Co³⁺, Rh⁺, Rh³⁺, Ir³⁺, Ni²⁺, Ni³⁺, Pd²⁺, Pt²⁺ und Pt⁴⁺, insbesondere Ru²⁺ und Ir³⁺,
Z = ausgewählt aus der Gruppe bestehend aus BF₄⁻, ClO₄⁻, SO₄²⁻, PO₄³⁻, F⁻, Cl⁻, Br⁻, I⁻, NO³⁻, PF₆⁻, O²⁻, OH⁻, CO₃²⁻, CN⁻, CF₃SO₃⁻, Cyanat, Isocyanat, Thiocyanat, Isothiocyanat, Tetraphenylborat und Acetat, insbesondere PF₆⁻.

4. Verbindung nach einem der Ansprüche 1 bis 3, **dadurch gekennzeichnet, dass** A = Wasserstoff und X = Sauerstoff, der Ligand also 2',6'-(Di(2:2"-pyridylcarbonyl)pyridin ist.

5. Verbindung nach einem der Ansprüche 1 bis 4, **dadurch gekennzeichnet, dass** die Übergangsmetalle Ruthenium in der Oxidationsstufe +2 oder Iridium in der Oxidationsstufe +3 sind.

6. Verbindung nach einem der Ansprüche 1 bis 5, **dadurch gekennzeichnet, dass** die Verbindung Bis[2',6'-di-(2:2"-pyridylcarbonyl)pyridin]ruthenium(II)-Hexafluorophosphat oder Bis[2',6'-di-(2:2"-pyridylcarbonyl)pyridin]iridium(III)-Hexafluorophosphat ist.

7. Verfahren zur Herstellung von Bis[2',6'-di-(2:2"-pyridylcarbonyl)pyridin]ruthenium(II)-Hexafluorophosphat oder Bis[2',6'-di-(2:2"-pyridylcarbonyl)pyridin]iridium(III)-Hexafluorophosphat umfassend die Schritte:
a) Auflösen von Ru(DMSO)₄Cl₂ oder Ir(DMSO)₄Cl₃ in Dimethylformamid,
b) Zugabe von 2,6-Bis(2-picolinyl)pyridin,
c) Erhitzen der Mischung für 240 bis 480 Minuten auf 90 bis 110°C,
d1) Abkühlen oder Abkühlen lassen, und
d2) Entfernung des Lösungsmittels, Aufreinigung des Reaktionsprodukts, durch Chromatographie, mit Acetonitril/Wasser/gesättigter KNO₃-Lösung im Volumenverhältnis 80:20:2 als Eluationsmittel, neuerliches Lösen des Feststoffs mit Methanol,
e) Versetzen der Lösung aus Schritt d2) mit wässriger Ammonium-Hexafluorophosphat-Lösung,
f) Abfiltrieren und Aufarbeiten des ausfallenden Niederschlags.

8. Verfahren zur Herstellung von Bis[2',6'-di-(2:2"-pyridylcarbonyl)pyridin]ruthenium(II)-Hexafluorophosphat
umfassend die Schritte:
a) Auflösen von Ru(DMSO)₄Cl₂ in Dimethylformamid,
b) Zugabe von 2,6-Bis(2-picolinyl)pyridin,
c) Erhitzen der Mischung für 10 Stunden auf 100°C,
d1) Abkühlen oder Abkühlen lassen, und
d2) Entfernung des Lösungsmittels, Aufreinigung des Reaktionsprodukts, durch Chromatographie, mit Acetonitril/Wasser/gesättigter KNO₃-Lösung im Volumenverhältnis 80:20:2 als Eluationsmittel, neuerliches Lösen des Feststoffs mit Methanol,
e) Versetzen der Lösung aus Schritt d2) mit wässriger Ammonium-Hexafluorophosphat-Lösung,
f) Abfiltrieren und Aufarbeiten des ausfallenden Niederschlags.

9. Verwendung von Verbindungen gemäß einem der Ansprüche 1 bis 6 in photovoltaischen Zellen bzw. Solarzellen, als Photokatalysator, als Sensibilisator für chemische Reaktionen, insbesondere in der Synthesechemie oder als Farbstoff.

## Claims

1. Compound of the general formula 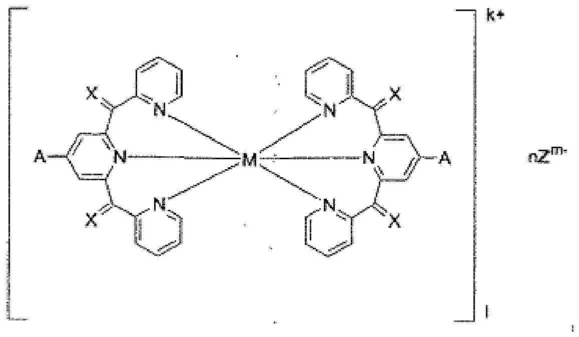 where
A is any type of substitution,
X is 0, S, NR, NH, PR, PH,
M is a transition metal having a charge k,
Z is a counterion having a negative charge m,
k is a charge k; non-negative whole number,
1 is a molecular equivalent; positive whole number, m is a negative charge m; positive whole number,
n is a molecular equivalent; non-negative whole number and with the proviso that the condition k*l=n*m is satisfied.

2. Compound according to Claim 1, **characterized in that** the variables have the following meanings
A = selected from the group consisting of organic substituents, -CF₃, -F, -Cl, -Br, -I, -OH, - SH, -NH₂, -NO₂ or -H, where the organic substituents are preferably selected from the group consisting of
- aryl residues, particularly phenyl, anthracenyl, pyrenyl,
- heteroaromatic residues, particularly pyridyl, nicotinyl, picolyl,
- unsaturated hydrocarbon residues, such as C₂-C₆-alkynyl, C₂-C₁₀-alkenyl, allyl, benzyl, and
- saturated hydrocarbon residues such as C₁-C₂₀-alkyl,
- -OR, -SR, -B(OR)₂, -COOR, -NHR, -NR₂, -CHR where R is aryl and C₁-C₂₀-alkyl, particularly phenyl, pyridyl or C₁-C₁₀-alkyl
X = 0, S, NR, NH, PR, PH, where R is aryl and C₁-C₂₀-alkyl, particularly phenyl or C₁-C₁₀-alkyl,
M = transition metal, in an oxidation state selected from the group consisting of 0, +1, +2, +3, +4, +5, +6, +7 and +8, preferably from the group consisting of +1, +2, +3, +4, particularly preferably selected from the group consisting of Cr³⁺, Mn²⁺, Re⁺, Re³⁺, Fe²⁺, Fe³⁺, R²⁺, R³⁺, O²⁺, Co²⁺, Co³⁺, Rh⁺, Rh³⁺, Ir³⁺, Ni²⁺, Ni³⁺, Pd²⁺, Pt²⁺ and Pt⁴⁺,
Z = counterion having a negative charge m, preferably selected from the group consisting of BF₄⁻, ClO₄⁻, SO₄²⁻, PO₄³⁻, F⁻, Cl⁻, Br⁻, I⁻, NO³⁻, PF₆⁻, O²⁻, OH⁻, CO₃²⁻, CN⁻, CF₃SO₃⁻, cyanate, isocyanate, thiocyanate, isothiocyanate, tetraphenylborate and acetate,
k having positive charge k; non-negative whole number k = 0, 1, 2, 3, 4, 5, 6, 7, 8, preferably 1, 2, 3, 4, particularly preferably 1, 2, 3.
l molecular equivalent; positive whole number
m having negative charge m; positive whole number m = 1, 2, 3, preferably 1,2, particularly preferably 1,
n molecular equivalent; non-negative whole number and
with the proviso that the condition k*l=n*m is satisfied.

3. Compound according to Claim 1 or 2, **characterized in that** the variables have the following meanings
A = benzyl-, phenyl-, ethynyl-, -F, -OH, -NH₂, -H, particularly hydrogen,
X = 0, S, NH, particularly 0,
M = selected from the group consisting of Cr³⁺, Mn²⁺, Re⁺, Re³⁺, Fe²⁺, Fe³⁺, Ru²⁺, Ru³⁺, Os²⁺, Co²⁺, Co³⁺, Rh⁺, Rh³⁺, Ir³⁺, Ni²⁺, Ni³⁺, Pd²⁺, Pt²⁺ and Pt⁴⁺, particularly Ru²⁺ and Ir³⁺,
Z = selected from the group consisting of BF₄⁻, ClO₄⁻, SO₄²⁻, PO₄³⁻, F⁻, Cl⁻, Br⁻, I⁻, NO³⁻, PF₆⁻, O²⁻, OH⁻, CO₃²⁻, CN⁻, CF₃SO₃⁻, cyanate, isocyanate, thiocyanate, isothiocyanate, tetraphenylborate and acetate, particularly PF₆⁻.

4. Compound according to any of Claims 1 to 3, **characterized in that** A = hydrogen and X = oxygen, the ligand therefore being 2',6'-(di(2:2"-pyridylcarbonyl)pyridine.

5. Compound according to any of Claims 1 to 4, **characterized in that** the transition metals are ruthenium in the +2 oxidation state or iridium in the +3 oxidation state.

6. Compound according to any of claims 1 to 5, **characterized in that** the compound is bis[2',6'-di(2:2"-pyridylcarbonyl)pyridine]ruthenium(II) hexafluorophosphate or bis[2',6'-di(2:2"-pyridylcarbonyl)pyridine]iridium(III) hexafluorophosphate.

7. Method for preparing bis[2',6'-di(2:2"-pyridylcarbonyl)pyridine]ruthenium(II) hexafluorophosphate or bis[2',6'-di(2:2"-pyridylcarbonyl)pyridine]iridium(III) hexafluorophosphate comprising the steps:
a) dissolving Ru(DMSO)₄Cl₂ or Ir(DMSO)₄Cl₃ in dimethylformamide
b) adding 2,6-bis(2-picolinyl)pyridine,
c) heating the mixture at 90 to 110°C for 240 to 480 minutes,
d1) cooling or allowing to cool, and
d2) removing the solvent, purifying the reaction product by chromatography with, as eluent, acetonitrile/water/saturated KNO₃ solution in the ratio 80:20:2 by volume, redissolving the solid with methanol,
e) treating the solution from step d2) with aqueous ammonium hexafluorophosphate solution,
f) filtering off and processing the precipitate which forms.

8. Method for preparing bis[2',6'-di(2:2"-pyridylcarbonyl)pyridine]ruthenium(II) hexafluorophosphate comprising the steps:
a) dissolving Ru(DMSO)₄Cl₂ in dimethylformamide
b) adding 2,6-bis(2-picolinyl)pyridine,
c) heating the mixture at 100°C for 10 hours,
d1) cooling or allowing to cool, and
d2) removing the solvent, purifying the reaction product by chromatography with, as eluent, acetonitrile/water/saturated KNO₃ solution in the ratio 80:20:2 by volume, redissolving the solid with methanol,
e) treating the solution from step d2) with aqueous ammonium hexafluorophosphate solution,
f) filtering off and processing the precipitate which forms.

9. Use of compounds according to any of Claims 1 to 6 in photovoltaic cells or solar cells, as photocatalysts, as sensitisers for chemical reactions, particularly in synthetic chemistry, or as dyes.

## Revendications

1. Composé de formule générale 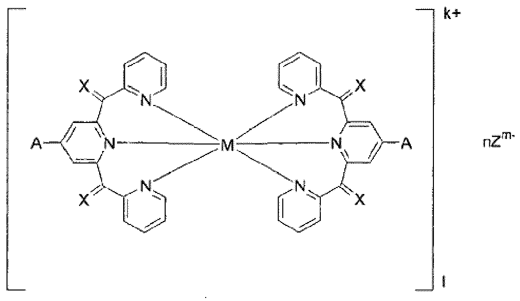 où
A représente tout type de substitution,
X représente 0, S, NR, NH, PR, PH,
M représente un métal de transition d'une charge k,
Z représente un contre-ion d'une charge négative m,
k représente la charge k ; un nombre entier non négatif,
l représente un équivalent moléculaire ; un nombre entier positif,
m représente la charge négative m ; un nombre entier positif,
n représente un équivalent moléculaire ; un nombre entier non négatif et
à condition que la condition k * l = n * m soit satisfaite.

2. Composé selon la revendication 1, **caractérisé en ce que** les variables ont les significations suivantes :
A = choisi dans le groupe constitué par les substituants organiques, -CF₃, -F, -Cl, -Br, -I, -OH, -SH, -NH₂, -NO₂ ou -H, les substituants organiques étant de préférence choisis dans le groupe constitué par
- les radicaux aryle, en particulier les radicaux phényle, anthracényle, pyrényle,
- les radicaux hétéroaromatiques, en particulier les radicaux pyridyle, nicotynyle, picolyle,
- les radicaux hydrocarbonés insaturés, tels que les radicaux C₂-C₆-alcynyle, C₂-C₁₀-alcényle, allyle, benzyle, et
- les radicaux hydrocarbonés saturés, tels que les radicaux C₁-C₂₀-alkyle,
- -OR, -SR, -B(OR)₂, -COOR, -NHR, -NR₂, -CHR R représentant aryle et C₁-C₂₀-alkyle, en particulier phényle, pyridyle ou C₁-C₁₀-alkyle
X = 0, S, NR, NH, PR, PH, R représentant aryle et C₁-C₂₀-alkyle, en particulier phényle ou C₁-C₁₀-alkyle,
M = un métal de transition, présentant un étage d'oxydation choisi dans le groupe constitué par 0, +1, +2, +3, +4, +5, +6, +7 et +8, de préférence dans le groupe constitué par +1, +2, +3 et +4, en particulier de préférence choisi dans le groupe constitué par Cr³⁺, Mn²⁺, Re⁺, Re³⁺, Fe²⁺, Fe³⁺, Ru²⁺, R³⁺, Os²⁺, Co²⁺, Co³⁺, Rh⁺, Rh³⁺, Ir³⁺, Ni²⁺, Ni³⁺, Pd²⁺, Pt²⁺ et Pt⁴⁺,
Z = contre-ion doté d'une charge négative m, de préférence choisi dans le groupe constitué par BF₄⁻, ClO₄⁻, SO₄²⁻, PO₄³⁻, F⁻, Cl⁻, Br⁻, I⁻, NO³⁻, PF₆⁻, O²⁻, OH⁻, CO₃²⁻, CN⁻, CF₃SO₃⁻, cyanate, isocyanate, thiocyanate, isothiocyanate, tétraphénylborate et acétate,
k charge positive k ; nombre entier non négatif k = 0, 1, 2, 3, 4, 5, 6, 7, 8, de préférence 1, 2, 3, 4, en particulier de préférence 1, 2, 3,
l un équivalent moléculaire ; un nombre entier positif,
m charge négative m ; nombre entier positif m = 1, 2, 3, de préférence 1, 2, en particulier de préférence 1,
n équivalent moléculaire ; un nombre entier non négatif et
à condition que la condition k * l = n * m soit satisfaite.

3. Composé selon la revendication 1 ou 2, **caractérisé en ce que** les variables ont les significations suivantes :
A = benzyle, phényle, éthynyle, -F, -OH, -NH₂, -H, en particulier hydrogène,
X = 0, S, NH, en particulier 0,
M = choisi dans le groupe constitué par Cr³⁺, Mn²⁺, Re⁺, Re³⁺, Fe²⁺, Fe³⁺, Ru²⁺, Ru³⁺, Os²⁺, Co²⁺, Co³⁺, Rh⁺, Rh³⁺, Ir³⁺, Ni²⁺, Ni³⁺, Pd²⁺, Pt²⁺ et Pt⁴⁺, en particulier Ru²⁺ et Ir³⁺,
Z = choisi dans le groupe constitué par BF₄⁻, ClO₄⁻, SO₄²-, PO₄³⁻, F⁻, Cl⁻, Br⁻, I⁻, NO³⁻, PF₆⁻, O²⁻, OH⁻, CO₃²⁻, CN⁻, CF₃SO₃⁻, cyanate, isocyanate, thiocyanate, isothiocyanate, tétraphénylborate et acétate, en particulier PF₆⁻.

4. Composé selon l'une quelconque des revendications 1 à 3, **caractérisé en ce que** A = hydrogène et X = oxygène, le ligand représentant donc 2',6'-(di(2:2"-pyridylcarbonyl)pyridine.

5. Procédé selon l'une quelconque des revendications 1 à 4, **caractérisé en ce que** les métaux de transition sont le ruthénium dans l'étage d'oxydation +2 ou l'iridium dans l'étage d'oxydation +3.

6. Composé selon l'une quelconque des revendications 1 à 5, **caractérisé en ce que** le composé est l'hexafluorophosphate de bis[2',6'-di-(2:2"-pyridylcarbonyl)pyridine]-ruthénium (II) ou l'hexafluorophosphate de bis[2',6'-di-(2:2"-pyridylcarbonyl)pyridine]-iridium (III).

7. Procédé pour la préparation d'hexafluorophosphate de bis[2',6'-di-(2:2"-pyridylcarbonyl)pyridine]-ruthénium (II) ou d'hexafluorophosphate de bis[2',6'-di-(2:2"-pyridylcarbonyl)pyridine]-iridium (III) comprenant les étapes :
a) dissolution de Ru(DMSO)₄Cl₂ ou d'Ir(DMSO)₄Cl₃ dans du diméthylformamide,
b) addition de 2,6-bis(2-picolinyl)pyridine,
c) chauffage du mélange pendant 240 à 480 minutes à 90 jusqu'à 110°C,
d1) refroidissement ou on laisse refroidir, et
d2) élimination du solvant, purification du produit de réaction par chromatographie avec un mélange acétonitrile/eau/solution saturée de KNO₃ dans un rapport volumique 80:20:2 comme éluant, redissolution du solide dans du méthanol,
e) ajout à la solution de l'étape d2) d'une solution aqueuse d'hexafluorophosphate d'ammonium,
f) filtration et traitement du précipité qui se sépare.

8. Procédé pour la préparation d'hexafluorophosphate de bis[2',6'-di-(2:2"-pyridylcarbonyl)pyridine]-ruthénium (II) comprenant les étapes :
a) dissolution de Ru(DMSO)₄Cl₂ dans du diméthylformamide,
b) addition de 2,6-bis(2-picolinyl)pyridine,
c) chauffage du mélange pendant 10 heures à 100°C,
d1) refroidissement ou on laisse refroidir, et
d2) élimination du solvant, purification du produit de réaction par chromatographie avec un mélange acétonitrile/eau/solution saturée de KNO₃ dans un rapport volumique 80:20:2 comme éluant, redissolution du solide dans du méthanol,
e) ajout à la solution de l'étape d2) d'une solution aqueuse d'hexafluorophosphate d'ammonium,
f) filtration et traitement du précipité qui se sépare.

9. Utilisation de composés selon l'une quelconque des revendications 1 à 6 dans des cellules photovoltaïques ou, selon le cas, des piles solaires, comme photocatalyseur, comme sensibilisateur pour des réactions chimiques, en particulier dans la chimie de synthèse, ou comme colorant.
